# EUROPEAN PATENT APPLICATION

(11) **EP 4 115 932 A1**
(43) Date of publication of application: **11.01.2023**
(21) Application number: 21183857.8
(22) Date of filing: 06.07.2021
(51) Int. Cl.: A61M 25/00, A61M 25/01

(54) **PRE-SHAPED MEDICAL CATHETERS**

(71) Applicant: ORBUSNEICH MEDICAL PTE. LTD, 079906 Singapore (SG)
(72) Inventor: COTTONE, Robert, DAVIE, 33330 (US); JUMAN, Mohamad Ike, DAVIE, 33331 (US)
(74) Representative: Jilderda, Anne Ayolt

(57) **Abstract**

A medical device (10), including a catheter body (12) defining a proximal segment (14), a distal segment (16) , and a lumen (18) therethrough, where the distal segment (16) includes a pre-shaped segment having a first geometric configuration in an unloaded state, and a second geometric configuration in a loaded state; where in the first geometric configuration, the pre-shaped segment (16) includes an arc of 180 degrees having a radius of at least 1 mm, and where a center of the arc is laterally offset from a longitudinal axis (20) of the proximal segment (14) of the catheter body (12).

## Description

### FIELD OF THE INVENTION

The present disclosure relates to intravascular medical devices and methods of use thereof.

### BACKGROUND OF THE INVENTION

Intravascular catheters are used in a variety of different treatment and diagnostic procedures to advance and position therapeutic devices to target locations within the body. Reaching such target locations may involve navigating tortuous vascular pathways having extreme turning or bending dimensions making access difficult for typical catheters. The present disclosure provides examples of pre-shaped vascular devices having sufficient flexibility and steerability to improve navigation and access to such tortuous pathways.

### FIELD OF THE INVENTION

The present disclosure advantageously provides a medical device, including a catheter body defining a proximal segment, a distal segment, and a lumen therethrough, wherein the distal segment includes a pre-shaped segment having a first geometric configuration in an unloaded state, and a second geometric configuration in a loaded state; wherein in the first geometric configuration, the pre-shaped segment includes an arc of 180 degrees having a radius of at least 1 mm, and wherein a center of the arc is laterally offset from a longitudinal axis of the proximal segment of the catheter body. The pre-shaped segment may include an arc of 180 degrees having a radius of at least 2 mm. The pre-shaped segment may transition from the first geometric configuration to the second geometric configuration under a load between 6 gF and 14 gF. The pre-shaped segment may transition from the first geometric configuration to the second geometric configuration under a load between 9 gF and 11 gF. At least a portion of the distal segment may be radiopaque. The distal segment may include an atraumatic distal tip.

A medical device is disclosed, including a catheter body defining a proximal segment, a distal segment, and a lumen therethrough, wherein the distal segment includes a pre-shaped segment having a first geometric configuration in an unloaded state, and a second geometric configuration in a loaded state; wherein in the first geometric configuration, the pre-shaped segment includes an arc of 180 degrees having a radius of at least 2 mm, and wherein a center of the arc is colinear with a longitudinal axis of the proximal segment of the catheter body. The catheter body may include an intermediate segment extending proximally of the distal segment, and the intermediate segment may define a radius of curvature between 20 mm and 30 mm. The intermediate segment may have a length between 5 mm and 15 mm. The intermediate segment may have an arc length between 30 degrees and 60 degrees. The pre-shaped segment may transition from the first geometric configuration to the second geometric configuration under a load between 6 gF and 14 gF.

Another medical device is provided, including a catheter body defining a proximal segment, a distal segment, and a lumen therethrough, wherein the distal segment includes a pre-shaped segment having a first geometric configuration in an unloaded state, and a second geometric configuration in a loaded state; wherein in the first geometric configuration, the pre-shaped segment includes an arc of 180 degrees having a radius of at least 2 mm, wherein a center of the arc is laterally offset from a longitudinal axis of the proximal segment of the catheter body, wherein the catheter body includes an intermediate segment extending proximally of the distal segment, and wherein the intermediate segment defines a radius of curvature between 20 mm and 30 mm. The intermediate segment may have a length between 10 mm and 20 mm. The intermediate segment may have an arc length between 45 degrees and 90 degrees. The pre-shaped segment may transition from the first geometric configuration to the second geometric configuration under a load between 6 gF and 14 gF.

A medical device is provided, including a catheter body defining a proximal segment, a distal segment, and a lumen therethrough, wherein the distal segment includes a non-linear pre-shaped segment having a first geometric configuration in an unloaded state, and a second geometric configuration in a loaded state; wherein the distal segment includes a tube with a cut pattern therein, the cut pattern defining: a plurality of cuts, with each cut having a width between 0.03 mm and 0.05 mm, and a longitudinal uncut width between 0.02 mm and 0.03 mm between each of the plurality of cuts; and wherein the pre-shaped segment transitions from the first geometric configuration to the second geometric configuration under a load between 6 gF and 14 gF. The cut pattern may define a strut height between 0.08 mm and 0.12 mm between each of the plurality of cuts. The cut pattern may define a pitch between 0.05 mm and 0.08 mm for each of the plurality of cuts.

In the first geometric configuration, the pre-shaped segment may include an arc of 180 degrees having a radius of at least 1 mm, and a center of the arc may be laterally offset from a longitudinal axis of the proximal segment of the catheter body. The pre-shaped segment may include an arc of 180 degrees having a radius of at least 2 mm.

In the first geometric configuration, the pre-shaped segment may include an arc of 180 degrees having a radius of at least 2 mm, and a center of the arc may be colinear with a longitudinal axis of the proximal segment of the catheter body.

In the first geometric configuration, the pre-shaped segment may include an arc of 180 degrees having a radius of at least 2 mm, and a center of the arc may laterally offset from a longitudinal axis of the proximal segment of the catheter body.

The pre-shaped segment may transition from the first geometric configuration to the second geometric configuration under a load between 9 gF and 11 gF. At least a portion of the distal segment may be radiopaque. The distal segment may include an atraumatic distal tip.

A medical device is provided, including a catheter body defining a proximal segment, a distal segment, and a lumen therethrough, wherein the distal segment includes a non-linear pre-shaped segment having a first geometric configuration in an unloaded state, and a second geometric configuration in a loaded state; wherein the distal segment includes a tube with a cut pattern therein, the cut pattern defining: a plurality of cuts, with each cut having a width between 0.03 mm and 0.05 mm, and a longitudinal uncut width between 0.01 mm and 0.03 mm between each of the plurality of cuts; and wherein the pre-shaped segment transitions from the first geometric configuration to the second geometric configuration under a load between 5 gF and 7 gF. The cut pattern may define a strut height between 0.06 mm and 0.15 mm between each of the plurality of cuts. The cut pattern may define a pitch between 0.05 mm and 0.07 mm for each of the plurality of cuts.

The catheter body may include a first tube segment proximal of the distal segment, the first tube segment having a cut pattern therein defining: a plurality of cuts forming an interrupted spiral cut, with each cut having a width between 0.03 mm and 0.05 mm, a longitudinal uncut width between 0.03 mm and 0.04 mm between each of the plurality of cuts, and a strut height between 0.08 mm and 0.12 mm between each of the plurality of cuts. The cut pattern of the first tube segment may define a pitch between 0.06 mm and 0.09 mm for each of the plurality of cuts.

The catheter body may include a second tube segment proximal of the first tube segment, the second tube segment having a cut pattern therein defining: a plurality of cuts forming an interrupted spiral cut, with each cut having a width between 0.03 mm and 0.05 mm, a longitudinal uncut width between 0.06 mm and 0.07 mm between each of the plurality of cuts, and a strut height between 0.08 mm and 0.12 mm between each of the plurality of cuts. The cut pattern of the second tube segment may define a pitch between 0.09 mm and 0.11 mm for each of the plurality of cuts.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings wherein:
FIG. 1A is a perspective view of a distal segment of an example of a medical device constructed in accordance with the principles of the present disclosure;
FIG. 1B is a side view the medical device shown in FIG. 1A;
FIG. 2A is a perspective view of a distal segment of an example of a medical device constructed in accordance with the principles of the present disclosure;
FIG. 2B is a side view the medical device shown in FIG. 2A;
FIG. 3A is a perspective view of a distal segment of an example of a medical device constructed in accordance with the principles of the present disclosure;
FIG. 3B is a side view the medical device shown in FIG. 3A;
FIG. 4A is a perspective view of a distal segment of an example of a medical device constructed in accordance with the principles of the present disclosure;
FIG. 4B is a side view the medical device shown in FIG. 4A;
FIG. 5 is a perspective view of a distal segment of an example of a medical device constructed in accordance with the principles of the present disclosure;
FIG. 6A is an illustration of an example of a portion of a catheter body construction in accordance with the principles of the present disclosure;
FIG. 6B is a closer view of the catheter body of FIG. 6A;
FIG. 6C is another illustration of the catheter body shown in FIG. 6A;
FIGS. 7A-6E illustrate an example of use of a medical device constructed in accordance with the principles of the present disclosure;
FIGS. 8A-8B illustrate an example of use of a medical device constructed in accordance with the principles of the present disclosure;
FIGS. 9A-9B illustrate an example of use of a medical device constructed in accordance with the principles of the present disclosure; and
FIG. 10 illustrates an example of a radiopaque coated region of an example of a medical device constructed in accordance with the principles of the present disclosure.

### DETAILED DESCRIPTION OF THE INVENTION

The present disclosure provides intravascular medical devices and methods of use thereof. In particular, the present disclosure provides pre-shaped medical devices that can aid in accessing and traversing vascular side branches or other vascular passages having sharp turns and/or small radii for accessibility.

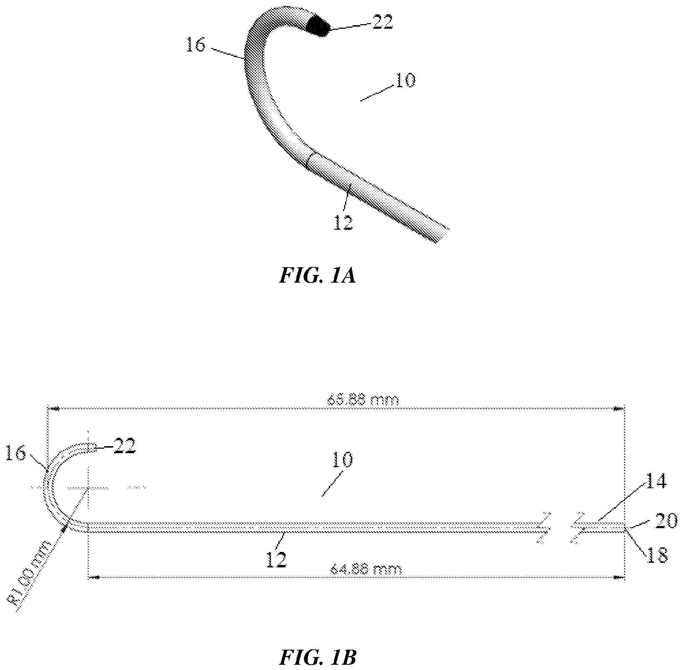

Now referring to the figures, FIGS. 1A-5B illustrate examples of an intravascular medical device 10, such as a catheter, constructed in accordance with the principles and advantages disclosed herein. The device 10 is a minimally-invasive device that can be introduced and operated intravenously in conjunction with one or more other devices as disclosed herein, such as those used in interventional cardiology to assess and/or treat occlusions or other vascular defects or conditions. The device 10 generally includes an elongated catheter body 12 with sufficient length, flexibility, and torqueability characteristics to be introduced and operated from an exterior of the patient, traverse the vasculature, and be positioned proximate the region being assessed or treated. The catheter body 12 generally includes a proximal segment 14 that may connect to and/or terminate at a hub or other component exterior to a patient (not shown), and a distal segment 16. The catheter body 12 further includes or defines a lumen 18 extending therethrough and exiting at the distal segment 16, where the lumen 18 has a diameter sufficient to pass a guidewire therethrough and/or to introduce one or more other medical instruments or devices through the catheter body 12.

Additional features of the device 10 and the catheter body 12 are provided in U.S. Patent Application Serial No. 15/726,024 (U.S. Pat. Pub. No. 2018/0093070), entitled 'MODULAR VASCULAR CATHETER,' and U.S. Patent Application Serial No. 16/255,141 (U.S. Pat. Pub. No. 2019/0160259), entitled 'VARIABLE FLEXIBILITY CATHETER SUPPORT FRAME,' entirety of all of which is incorporated herein by reference.

The distal segment 16 of the device 10 may include one or more pre-shaped segments to provide a desired geometry to aid in accessing or traversing certain vascularity. The device 10 may include or define the one or more pre-shaped segments when in a 'neutral' or unloaded configuration/state, and may be transitionable into another geometric configuration/state or shape when placed under a load or strain of a particular threshold force level that may include, for example, routing one or more medical devices or instruments through the device 10, as described herein.

In the example shown in FIGS. 1A-1B, the distal segment 16 defines a substantially semicircular shape that has a center of radius "R" that is offset from a longitudinal axis 20 of a substantially linear portion of the catheter body 12. This pre-shaped distal segment 16 may define a radius of approximately 1 mm (+/- 15%), and/or may provide an arc length of approximately 180 degrees (+/- 15%).

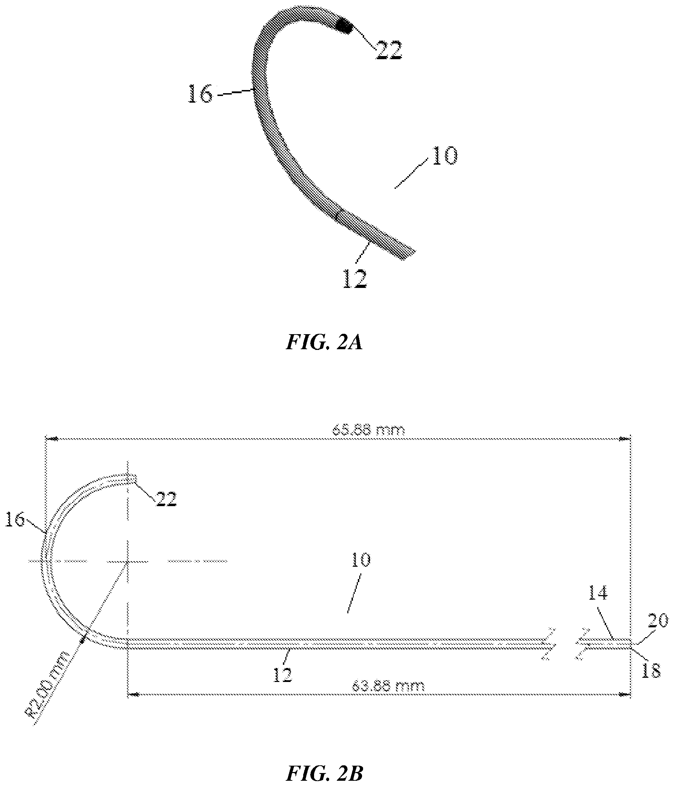

In the example shown in FIGS. 2A-2B, the distal segment 16 defines a substantially semicircular shape that has a center of radius "R" that is offset from the longitudinal axis 20 of a substantially linear portion of the catheter body 12. This pre-shaped distal segment 16 may define a radius of approximately 2 mm (+/- 15%), and/or may provide an arc length of approximately 180 degrees (+/- 15%).

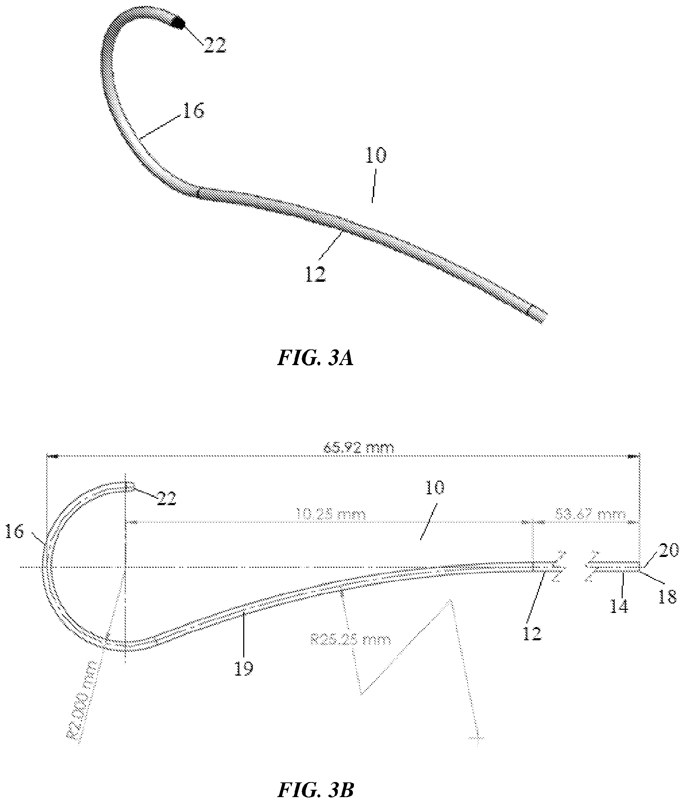

In the example shown in FIGS. 3A-3B, the distal segment 16 defines a substantially semicircular shape that has a center of radius "R" that is substantially coaxial with the longitudinal axis 20 of a substantially linear portion of the catheter body 12. This pre-shaped distal segment 16 may define a radius of approximately 2 mm (+/- 15%), and/or may provide an arc length of approximately 180 degrees (+/- 15%). The device 10 may include an intermediate segment 19 that is curved or at least partially offset from the longitudinal axis 20 to provide the substantially colinear alignment between the center of curvature of the segment 16 and the longitudinal axis 20. The intermediate segment 19 may have for example, a length between approximately 5 mm and approximately 15 mm, may define a radius of curvature between approximately 20 mm and approximately 30 mm, and/or may have an arc length between approximately 30 degrees and approximately 60 degrees.

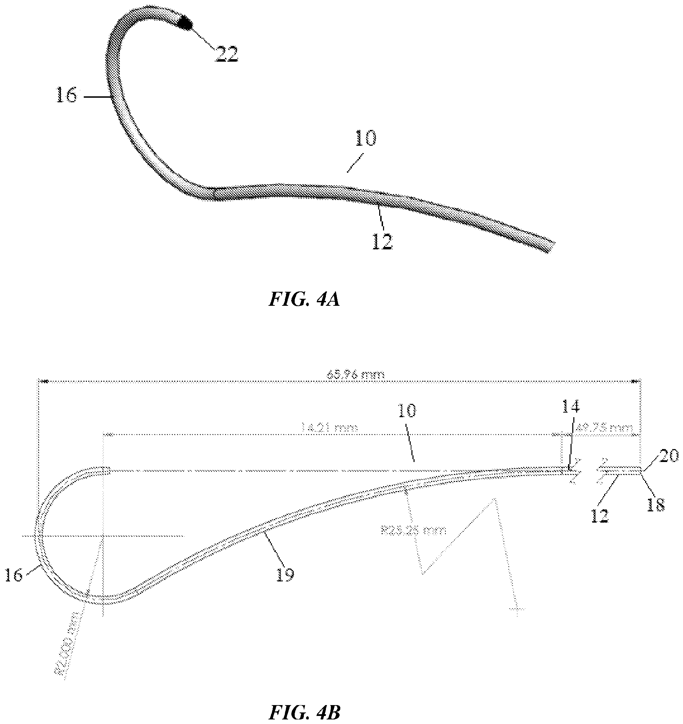

In the example shown in FIGS. 4A-4B, the distal segment 16 defines a substantially semicircular shape that has a center of radius "R" that is offset from the longitudinal axis 20 of a substantially linear portion of the catheter body 12 such that the distal-most tip or end 22 of the device 10 is substantially colinear or coaxial with the longitudinal axis 20. This pre-shaped distal segment 16 may define a radius of approximately 2 mm (+/- 15%), and/or may provide an arc length of approximately 180 degrees (+/- 15%). The device 10 may include an intermediate segment 19 that is curved or at least partially offset from the longitudinal axis 20 to provide the substantially colinear alignment between the tip 22 and the longitudinal axis 20. The intermediate segment 19 may have for example, a length between approximately 10 mm and approximately 20 mm, may define a radius of curvature between approximately 20 mm and approximately 30 mm, and/or may have an arc length between approximately 45 degrees and approximately 90 degrees.

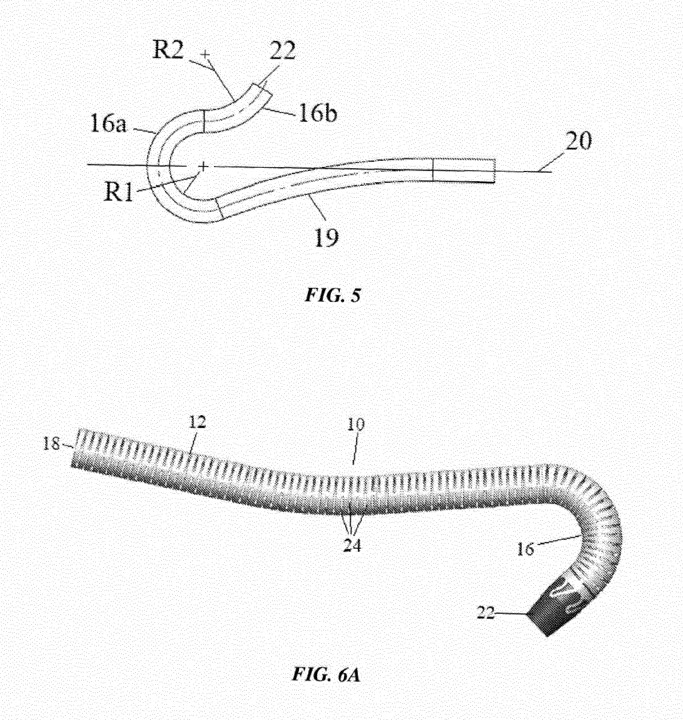

In the example shown in FIG. 5, the distal segment 16 has a first segment 16a that defines a substantially semicircular shape that has a center of radius "R" that is substantially coaxial with the longitudinal axis 20 of a substantially linear portion of the catheter body 12. This pre-shaped first segment 16a may define a radius R1 of approximately 0.5 - 3 mm, preferably 2 mm, (+/-15%), and/or may provide an arc length of approximately 180 degrees (+/- 15%). The distal segment 16 may also include a second segment 16b extending from the first segment 16a, where the second segment 16b defines an arcuate shape that may define a radius R2 of approximately 0.25 mm ― 6 mm, preferably 3 mm, (+/- 15%), and/or may provide an arc length of approximately 10 degrees to 60 degrees (+/- 15%). The device 10 may include an intermediate segment 19 that is curved or at least partially offset from the longitudinal axis 20 to provide the substantially colinear alignment between the center of curvature of the segment 16a and the longitudinal axis 20. The intermediate segment 19 may have for example, a length between approximately 5 mm and approximately 15 mm, may define a radius of curvature between approximately 20 mm and approximately 30 mm, and/or may have an arc length between approximately 30 degrees and approximately 60 degrees.

The examples and associated dimensions of the medical devices 10 described above may be modified for different applications to conform to and/or adapt for use in different anatomical structures and/or for different patients.

The pre-shaped segment(s) described herein may be constructed in various ways to provide the disclosed geometrical configurations and variations thereof. For example, the distal segments may be constructed primarily or partially from shape-memory alloys, such as nickel-titanium alloys or other materials, and heat treating one or more portions of the device may provide one or more varying degrees of flexibility and/or set shape along the length of the device 10 as desired. Such construction may also and/or alternatively include, for example, cutting one or more patterns into a metal tube to provide the catheter body 12 one or more varying degrees of flexibility and/or set shape along the length of the device 10 as desired.

The medical device 10 may include sufficient stiffness along its length to substantially resist axial compression and elastic deformity when under forces or loads accompanying intravascular or other medical treatments and procedures. The pre-shaped or geometrically-biased configurations of the device 10 is also transitionable from its primary pre-shaped configuration when in a 'neutral' or unloaded state/configuration to a secondary, changed geometric configuration or shape when placed under a load or strain of a particular threshold force level. The threshold force level may include, for example, a force experienced by the medical device 10 when routing one or more medical devices or instruments through the device 10. In one example, the pre-shaped configuration of the medical device 10 may be overcome by routing a guidewire through the lumen 18 of the device to cause the device 10 to take on the contour (or lack thereof) of the guidewire extending therethrough. The pre-shaped segment(s) of the medical device 10 may be sufficiently flexible such that a guidewire routed through the device 10 can overcome the pre-shaped configuration, yet the pre-shaped segment(s) must be sufficiently stiff to be able to navigate a sharp turn or othered tortuous anatomy of a patient through manipulation of the proximal end of the device 10 by a physician.

In one example, the pre-shaped segment(s) of the medical device 10 may have a shape-retention threshold between approximately 6 gram Force (gF) and approximately 14 gF, where a force or load exceeding these levels causes the pre-shaped segment to change shape, i.e., transition from the pre-shaped configuration to a substantially linear shape or otherwise.

The desired stiffness of the medical device may be achieved by modifying one or more characteristics of the device, including for example, wall thickness, cut patterns, or the like.

The cut pattern of the catheter body and the pre-shaped segment(s) may include a substantially uniform plurality of cuts 24 along one or more lengths or zones thereof, examples of which are shown in FIGS. 6A-6B. The cut pattern may include a cut width 26, a longitudinal uncut width 28 between cut segments, a strut height 30 (e.g., interrupted, uncut length between consecutive partially spiral cuts) between the cuts to form an interrupted spiral configuration and/or to otherwise connect the uncut portions of the pattern, and a pitch distance 32 (e.g., the length along the longitudinal axis of the catheter body 12 that a cut traverses to complete one revolution around the circumference of the catheter body).

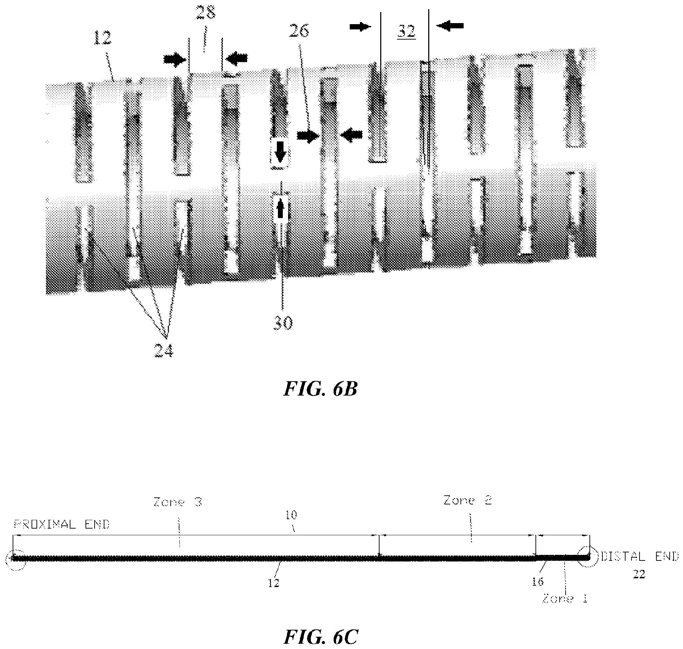

Now referring to FIG. 6C, the catheter body 12 of the medical device 10 may include one or more zones that include variations in the plurality of cuts along the length of the catheter body 12 to vary the flexibility of the device. For example, the catheter body may include a first zone ("Zone 1") that includes the distal segment 16 and the distal end 22. A second zone ("Zone 2") may extend proximally from Zone 1, and a third zone ("Zone 3") may extend from Zone 2 and encompass the proximal segment 14. The zones of the catheter body 12 may provide an increase in flexibility from the proximal segment 14 to the distal segment 16, and each zone may include variations in the characteristics of the plurality of cuts (e.g., cut width, uncut width, pitch, etc.) compared to the other zones to provide the desired torqueability, flexibility, and pre-shaped characteristics described herein.

In one example, the medical device 10 may include a catheter body 12 with a first zone having the plurality of cuts 24 characterized by an interrupted spiral cut with a cut width 26 between approximately 0.03 mm and approximately 0.05 mm, and may preferably be approximately 0.04 mm. The plurality of cuts 24 in the first zone may include an uncut width 28 between approximately 0.02 mm and approximately 0.03 mm, and may preferably be approximately 0.024 mm. The plurality of cuts 24 in the first zone may include a strut height 30 between approximately 0.08 mm and approximately 0.12 mm, and may preferably be approximately 0.105 mm. The plurality of cuts 24 in the first zone may include a pitch 32 between approximately 0.05 mm and approximately 0.08 mm, and may preferably be approximately 0.064 mm. This first zone may have a shape-retention threshold between approximately 9 gF and approximately 11 gF.

Continuing in this example of an exemplary medical device, a second zone having the plurality of cuts 24 may be characterized by an interrupted spiral cut with a cut width 26 between approximately 0.03 mm and approximately 0.05 mm, and may preferably be approximately 0.04 mm. The plurality of cuts 24 in the second zone may include an uncut width 28 between approximately 0.03 mm and approximately 0.04 mm, and may preferably be approximately 0.036 mm. The plurality of cuts 24 in the second zone may include a strut height 30 between approximately 0.08 mm and approximately 0.12 mm, and may preferably be approximately 0.105 mm. The plurality of cuts 24 in the first zone may include a pitch 32 between approximately 0.06 mm and approximately 0.09 mm, and may preferably be approximately 0.076 mm.

A third zone having the plurality of cuts 24 may be characterized by an interrupted spiral cut with a cut width 26 between approximately 0.03 mm and approximately 0.05 mm, and may preferably be approximately 0.04 mm. The plurality of cuts 24 in the third zone may include an uncut width 28 between approximately 0.06 mm and approximately 0.07 mm, and may preferably be approximately 0.062 mm. The plurality of cuts 24 in the third zone may include a strut height 30 between approximately 0.08 mm and approximately 0.12 mm, and may preferably be approximately 0.105 mm. The plurality of cuts 24 in the third zone may include a pitch 32 between approximately 0.09 mm and approximately 0.11 mm, and may preferably be approximately 0.102 mm.

In another example, the medical device 10 may include a catheter body 12 with a first zone having the plurality of cuts 24 characterized by an interrupted spiral cut with a cut width 26 between approximately 0.03 mm and approximately 0.05 mm, and may preferably be approximately 0.04 mm. The plurality of cuts 24 in the first zone may include an uncut width 28 between approximately 0.01 mm and approximately 0.03 mm. The plurality of cuts 24 in the first zone may include a strut height 30 between approximately 0.06 mm and approximately 0.15 mm. The plurality of cuts 24 in the first zone may include a pitch 32 between approximately 0.05 mm and approximately 0.07 mm. This first zone may have a shape-retention threshold between approximately 5 gF and approximately 7 gF.

Continuing in this example of an exemplary medical device, a second zone having the plurality of cuts 24 may be characterized by an interrupted spiral cut with a cut width 26 between approximately 0.03 mm and approximately 0.05 mm, and may preferably be approximately 0.04 mm. The plurality of cuts 24 in the second zone may include an uncut width 28 between approximately 0.03 mm and approximately 0.04 mm, and may preferably be approximately 0.036 mm. The plurality of cuts 24 in the second zone may include a strut height 30 between approximately 0.08 mm and approximately 0.12 mm, and may preferably be approximately 0.105 mm. The plurality of cuts 24 in the first zone may include a pitch 32 between approximately 0.06 mm and approximately 0.09 mm, and may preferably be approximately 0.076 mm.

A third zone having the plurality of cuts 24 may be characterized by an interrupted spiral cut with a cut width 26 between approximately 0.03 mm and approximately 0.05 mm, and may preferably be approximately 0.04 mm. The plurality of cuts 24 in the third zone may include an uncut width 28 between approximately 0.06 mm and approximately 0.07 mm, and may preferably be approximately 0.062 mm. The plurality of cuts 24 in the third zone may include a strut height 30 between approximately 0.08 mm and approximately 0.12 mm, and may preferably be approximately 0.105 mm. The plurality of cuts 24 in the third zone may include a pitch 32 between approximately 0.09 mm and approximately 0.11 mm, and may preferably be approximately 0.102 mm.

In another example, the medical device 10 may include a catheter body 12 with a first zone having the plurality of cuts 24 characterized by an interrupted spiral cut with a cut width 26 between approximately 0.03 mm and approximately 0.05 mm, and may preferably be approximately 0.04 mm. The plurality of cuts 24 in the first zone may include an uncut width 28 between approximately 0.01 mm and approximately 0.03 mm. The plurality of cuts 24 in the first zone may include a strut height 30 between approximately 0.059 mm and approximately 0.14 mm. The plurality of cuts 24 in the first zone may include a pitch 32 between approximately 0.05 mm and approximately 0.07 mm. This first zone may have a shape-retention threshold between approximately 6 gF and approximately 8 gF.

Continuing in this example of an exemplary medical device, a second zone having the plurality of cuts 24 may be characterized by an interrupted spiral cut with a cut width 26 between approximately 0.03 mm and approximately 0.05 mm, and may preferably be approximately 0.04 mm. The plurality of cuts 24 in the second zone may include an uncut width 28 between approximately 0.03 mm and approximately 0.04 mm, and may preferably be approximately 0.036 mm. The plurality of cuts 24 in the second zone may include a strut height 30 between approximately 0.08 mm and approximately 0.12 mm, and may preferably be approximately 0.105 mm. The plurality of cuts 24 in the first zone may include a pitch 32 between approximately 0.06 mm and approximately 0.09 mm, and may preferably be approximately 0.076 mm.

A third zone having the plurality of cuts 24 may be characterized by an interrupted spiral cut with a cut width 26 between approximately 0.03 mm and approximately 0.05 mm, and may preferably be approximately 0.04 mm. The plurality of cuts 24 in the third zone may include an uncut width 28 between approximately 0.06 mm and approximately 0.07 mm, and may preferably be approximately 0.062 mm. The plurality of cuts 24 in the third zone may include a strut height 30 between approximately 0.08 mm and approximately 0.12 mm, and may preferably be approximately 0.105 mm. The plurality of cuts 24 in the third zone may include a pitch 32 between approximately 0.09 mm and approximately 0.11 mm, and may preferably be approximately 0.102 mm.

The device 10 may include a distal tip having a rounded and/or tapered atraumatic profile that may be constructed from a relatively soft or pliable material. One or more portions of the device 10 may be radiopaque and/or include radiopaque markers to aid in medical imaging of the device during a procedure. Radiopaque features may be achieved, for example, through the inclusion or infusion of tungsten, bismuth, and/or barium sulphate into one or more components of the device. The device 10 may also include one or more polymer liners or coating on surfaces thereof along the length of the device.

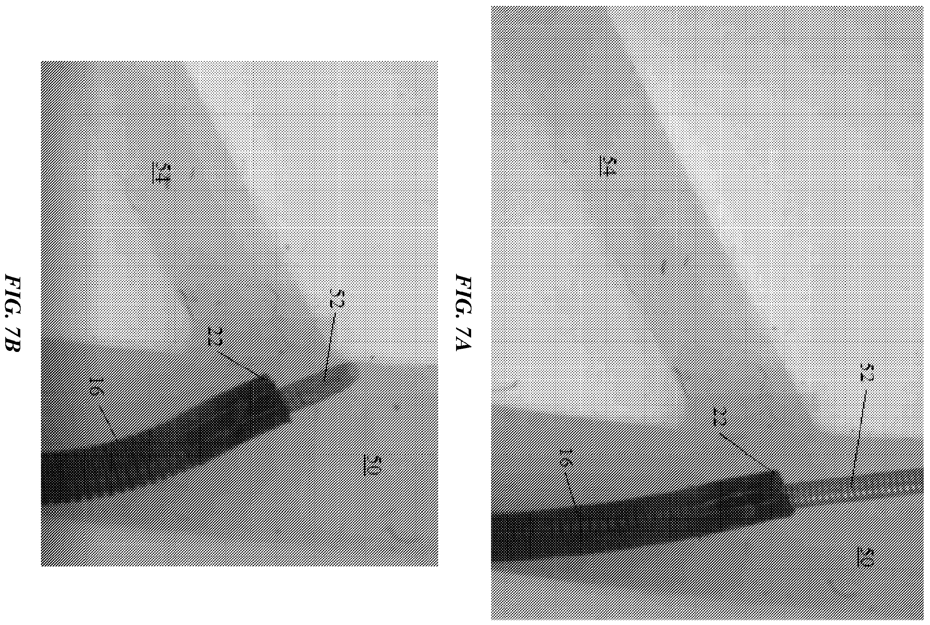

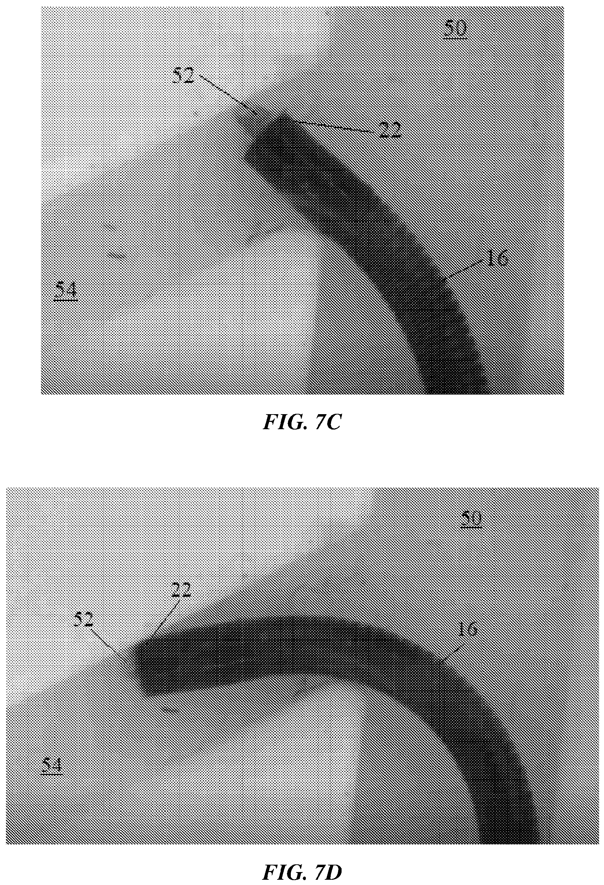

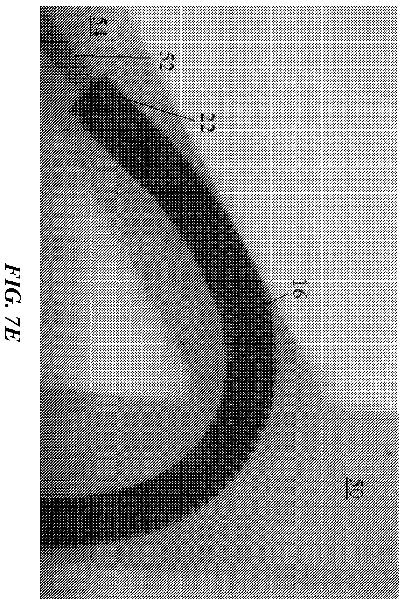

Now referring to FIGS. 7A-7E, an exemplary method of use of the device 10 is shown. For example, FIG. 6A shows the distal segment 16 of the device 10 positioned in a primary pathway 50, which may include a vascular segment or other physiological path. A guidewire 52 is routed through the lumen 18 of medical device 10 and extends from the distal end of the device 10. The stiffness of the guidewire 52 is sufficient to overcome the pre-shaped, biased geometric configuration of the medical device 10 such that the distal segment 16 of the device 10 contours to the curvature (or lack thereof) of the guidewire.

The device 10 and the guidewire 52 may then be manipulated to access a second pathway 54, which may include a vascular segment or other physiological path, having a small radius or other angled approach that the guidewire otherwise would be unable to access due to limitations on the ability to manipulate the guidewire 52 into a "U"-turn or other sharp corner that may exceed 90 degrees.

Upon positioning the device 10 and the guidewire 52 in proximity to the second pathway 54 for further routing or access of the guidewire and medical device, the guidewire 52 may be retracted a sufficient distance within the device 10 to allow the pre-shaped distal segment 16 of the device 10 to regain its shape, as shown in FIGS. 6B-6D. The pre-shaped curvature of the distal segment 16 can then be positioned into the second pathway 54 a sufficient distance to allow extending the guidewire from the device 10 and into the second pathway 54, as shown in FIG. 6E. The device 10 may then be removed from the guidewire 52 and other devices may be routed along the guidewire 52 and into the second pathway, and/or other devices may be routed through the lumen 18 of the device 10 and into the second pathway 54 for subsequent use.

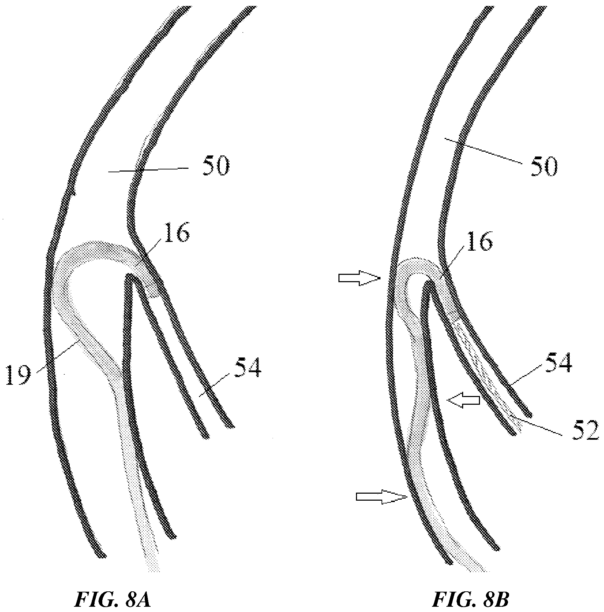

Now referring to FIGS. 8A-8B, an exemplary method of use of the example of the device 10 shown in FIGS. 3A-4B is shown. In the illustrated example, the intermediate segment 19 of the device 10 and the geometry of the distal segment 16 facilitates navigating to and accessing a secondary vascular path/branch 54 having a small radius or other angled approach, as shown in FIG. 8A. Once the distal-most end 22 is positioned in or near the secondary pathway 54, the guidewire 52 can be extended through the catheter body 12 and into the secondary pathway 54, as shown in FIG. 8B. An axial force directed along the catheter body 12 while extending the guidewire 52 can shift the shape and/or position of the device 10 such that portions of the intermediate segment and/or the distal segment 16 abut the surrounding vasculature wall of the primary path 50 and the secondary path 54 as indicated by the arrows in FIG. 8B. This contact provides anchoring backup support to the position of the distal segment 16 such that the movement of the guidewire 52 out of the distal end 22 and into the secondary path 54 does not cause the distal segment 16 to back out of the secondary path 54 and lose position. Instead, the anchoring of the device 10 due to the pre-shaped segment resists any backout force imparted by the guidewire 52 being introduced into the second path 54.

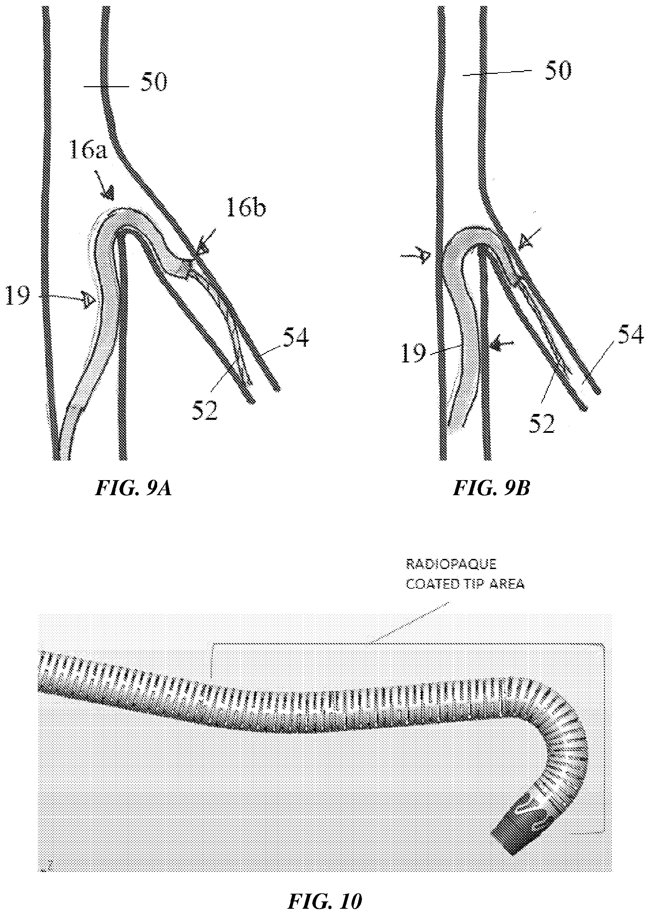

FIGS. 9A-9B illustrate an exemplary method of use of the example of the device 10 shown in FIG. 5. Similar to the resistance provided to backing out of the device from a secondary path 54 as described above, in the illustrated example of FIG. 9A, the intermediate segment 19 of the device 10 and the geometry of the distal segment 16 facilitates navigating to and accessing a secondary vascular path/branch 54 having a small radius or other angled approach. Once the distal-most end 22 is positioned in or near the secondary pathway 54, the guidewire 52 can be extended through the catheter body 12 and into the secondary pathway 54, as shown in FIG. 9B. An axial force directed along the catheter body 12 while extending the guidewire 52 can shift the shape and/or position of the device 10 such that portions of the intermediate segment and/or the distal segment 16 abut the surrounding vasculature wall of the primary path 50 and the secondary path 54 as indicated by the arrows in FIG. 9B. This contact provides anchoring backup support to maintain and secure the position of the distal segment 16.

The medical device 10 may include one or more coatings, liners, and/or other layered components disposed on interior and/or exterior surfaces thereof. For example, the distal segment 16 may include a radiopaque coating to improve the visibility of the device 10 under medical imaging means, an example of which is shown in FIG. 10. Such radiopaque coating may include, for example, a layer or coating of gold, platinum, tungsten, iridium, or the like in a thickness between approximately 1 to 100 microns. The device 10 may include one or more lubricious coatings or layers on interior and/or exterior surfaces thereof. The device 10 may include a polymer liner or coating on a portion of the interior lumen 18, where the liner terminates or otherwise does not extend into the pre-shaped distal segment 16 to avoid adversely affecting the flexibility of the distal segment 16 and/or the ability of the distal segment 16 to retain its pre-shaped configuration.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

### Specific Embodiments of the Invention:

The present invention particularly encompasses the following specific embodiments:
1. A medical device, comprising: a catheter body defining a proximal segment, a distal segment, and a lumen there through, wherein the distal segment includes a pre-shaped segment having a first geometric configuration in an unloaded state, and a second geometric configuration in a loaded state; wherein in the first geometric configuration, the pre-shaped segment includes an arc of 180 degrees having a radius of at least 1 mm, and wherein a center of the arc is laterally offset from a longitudinal axis of the proximal segment of the catheter body.
2. The medical device of embodiment 1, wherein the pre-shaped segment includes an arc of 180 degrees having a radius of at least 2 mm.
3. The medical device of embodiment 1, wherein the pre-shaped segment transitions from the first geometric configuration to the second geometric configuration under a load between 6 gF and 14 gF.
4. The medical device of embodiment 1, wherein the pre-shaped segment transitions from the first geometric configuration to the second geometric configuration under a load between 9 gF and 11 gF.
5. The medical device of embodiment 1, wherein at least a portion of the distal segment is radiopaque.
6. The medical device of embodiment 1, wherein the distal segment includes an atraumatic distal tip.
7. A medical device, comprising: a catheter body defining a proximal segment, a distal segment, and a lumen there through, wherein the distal segment includes a pre-shaped segment having a first geometric configuration in an unloaded state, and a second geometric configuration in a loaded state; wherein in the first geometric configuration, the pre-shaped segment includes an arc of 180 degrees having a radius of at least 2 mm, and wherein a center of the arc is colinear with a longitudinal axis of the proximal segment of the catheter body.
8. The medical device of embodiment 7, wherein the catheter body includes an intermediate segment extending proximally of the distal segment, and wherein the intermediate segment defines a radius of curvature between 20 mm and 30 mm.
9. The medical device of embodiment 8, wherein the intermediate segment has a length between 5 mm and 15mm.
10. The medical device of embodiment 8, wherein the intermediate segment has an arc length between 30 degrees and 60 degrees.
11. The medical device of embodiment 7, wherein the pre-shaped segment transitions from the first geometric configuration to the second geometric configuration under a load between 6 gF and 14 gF.
12. A medical device, comprising: a catheter body defining a proximal segment, a distal segment, and a lumen there through, wherein the distal segment includes a pre-shaped segment having a first geometric configuration in an unloaded state, and a second geometric configuration in a loaded state; wherein in the first geometric configuration, the pre-shaped segment includes an arc of 180 degrees having a radius of at least 2 mm, wherein a center of the arc is laterally offset from a longitudinal axis of the proximal segment of the catheter body, wherein the catheter body includes an intermediate segment extending proximally of the distal segment, and wherein the intermediate segment defines a radius of curvature between 20 mm and 30 mm.
13. The medical device of embodiment 12, wherein the intermediate segment has a length between 10 mm and 20 mm.
14. The medical device of embodiment 12, wherein the intermediate segment has an arc length between 45 degrees and 90 degrees.
15. The medical device of embodiment 12, wherein the pre-shaped segment transitions from the first geometric configuration to the second geometric configuration under a load between 6 gF and 14 gF.
16. A medical device, comprising: a catheter body defining a proximal segment, a distal segment, and a lumen there through, wherein the distal segment includes a non-linear pre-shaped segment having a first geometric configuration in an unloaded state, and a second geometric configuration in a loaded state; wherein the distal segment includes a tube with a cut pattern therein, the cut pattern defining: a plurality of cuts, with each cut having a width between 0.03 mm and 0.05 mm, and a longitudinal uncut width between 0.02 mm and 0.03 mm between each of the plurality of cuts; and wherein the pre-shaped segment transitions from the first geometric configuration to the second geometric configuration under a load between 6 gF and 14 gF.
17. The medical device of embodiment 16, wherein the cut pattern defines a strut height between 0.08 mm and 0.12 mm between each of the plurality of cuts.
18. The medical device of embodiment 17, wherein the cut pattern defines a pitch between 0.05 mm and 0.08 mm for each of the plurality of cuts.
19. The medical device of embodiment 16, wherein in the first geometric configuration, the pre-shaped segment includes an arc of 180 degrees having a radius of at least 1 mm, and wherein a center of the arc is laterally offset from a longitudinal axis of the proximal segment of the catheter body.
20. The medical device of embodiment 19, wherein the pre-shaped segment includes an arc of 180 degrees having a radius of at least 2 mm.
21. The medical device of embodiment 16, wherein in the first geometric configuration, the pre-shaped segment includes an arc of 180 degrees having a radius of at least 2 mm, and wherein a center of the arc is colinear with a longitudinal axis of the proximal segment of the catheter body.
22. The medical device of embodiment 16, wherein in the first geometric configuration, the pre-shaped segment includes an arc of 180 degrees having a radius of at least 2 mm, and wherein a center of the arc is laterally offset from a longitudinal axis of the proximal segment of the catheter body.
23. The medical device of embodiment 16, wherein the pre-shaped segment transitions from the first geometric configuration to the second geometric configuration under a load between 9 gF and 11 gF.
24. The medical device of embodiment 16, wherein at least a portion of the distal segment is radiopaque.
25. The medical device of embodiment 16, wherein the distal segment includes an atraumatic distal tip.
26. A medical device, comprising: a catheter body defining a proximal segment, a distal segment, and a lumen there through, wherein the distal segment includes a non-linear pre-shaped segment having a first geometric configuration in an unloaded state, and a second geometric configuration in a loaded state; wherein the distal segment includes a tube with a cut pattern therein, the cut pattern defining: a plurality of cuts, with each cut having a width between 0.03 mm and 0.05 mm, and a longitudinal uncut width between 0.01 mm and 0.03 mm between each of the plurality of cuts; and wherein the pre-shaped segment transitions from the first geometric configuration to the second geometric configuration under a load between 5 gF and 7 gF.
27. The medical device of embodiment 26, wherein the cut pattern defines a strut height between 0.06 mm and 0.15 mm between each of the plurality of cuts.
28. The medical device of embodiment 27, wherein the cut pattern defines a pitch between 0.05 mm and 0.07 mm for each of the plurality of cuts.
29. The medical device of embodiment 26, wherein the catheter body includes a first tube segment proximal of the distal segment, the first tube segment having a cut pattern therein defining: a plurality of cuts forming an interrupted spiral cut, with each cut having a width between 0.03 mm and 0.05 mm, a longitudinal uncut width between 0.03 mm and 0.04 mm between each of the plurality of cuts, and a strut height between 0.08 mm and 0.12 mm between each of the plurality of cuts.
30. The medical device of embodiment 29, wherein the cut pattern of the first tube segment defines a pitch between 0.06 mm and 0.09 mm for each of the plurality of cuts.
31. The medical device of embodiment 26, wherein the catheter body includes a second tube segment proximal of the first tube segment, the second tube segment having a cut pattern therein defining: a plurality of cuts forming an interrupted spiral cut, with each cut having a width between 0.03 mm and 0.05 mm, a longitudinal uncut width between 0.06 mm and 0.07 mm between each of the plurality of cuts, and a strut height between 0.08 mm and 0.12 mm between each of the plurality of cuts.
32. The medical device of embodiment 31, wherein the cut pattern of the second tube segment defines a pitch between 0.09 mm and 0.11 mm for each of the plurality of cuts.

## Claims

1. A medical device, comprising: a catheter body defining a proximal segment, a distal segment, and a lumen there through, wherein the distal segment includes a pre-shaped segment having a first geometric configuration in an unloaded state, and a second geometric configuration in a loaded state; wherein in the first geometric configuration, the pre-shaped segment includes an arc of 180 degrees having a radius of at least 1 mm, particularly of at least 2 mm, and wherein a centre of the arc is laterally offset from a longitudinal axis of the proximal segment of the catheter body.

2. The medical device of claim 1, wherein the pre-shaped segment transitions from the first geometric configuration to the second geometric configuration under a load between 6 gF and 14 gF, particularly between 9 gF and 11 gF.

3. The medical device of claim 1 or 2, wherein at least a portion of the distal segment is radiopaque.

4. The medical device of claim 1, 2 or 3, wherein the distal segment includes an atraumatic distal tip.

5. A medical device according to any of the preceding claims, wherein a centre of the arc is colinear with a longitudinal axis of the proximal segment of the catheter body.

6. The medical device of claim 5, wherein the catheter body includes an intermediate segment extending proximally of the distal segment, and wherein the intermediate segment defines a radius of curvature between 10 mm and 30 mm, particularly between 10 mm and 20 mm, more particularly between 20 and 30 mm.

7. The medical device of claim 6, wherein the intermediate segment has a length between 5 mm and 15mm.

8. The medical device of claim 6 or 7, wherein the intermediate segment has an arc length between 30 degrees and 60 degrees, particularly between 45 degrees and 90 degrees.

9. The medical device according to anyone of the preceding claims, **characterized in that** the distal segment includes a tube with a cut pattern therein, the cut pattern defining: a plurality of cuts, with each cut having a width between 0.03 mm and 0.05 mm, and a longitudinal uncut with.

10. The medical device as claimed in claim 9, wherein the longitudinal uncut width is between 0.02 mm and 0.03 mm between each of the plurality of cuts, and wherein the pre-shaped segment transitions from the first geometric configuration to the second geometric configuration under a load between 6 gF and 14 gF, particularly between 9 gF and 11 gF. [16]

11. The medical device of claim 10, wherein the cut pattern defines a strut height between 0.08 mm and 0.12 mm between each of the plurality of cuts.

12. The medical device of claim 11 or 12, wherein the cut pattern defines a pitch between 0.05 mm and 0.08 mm for each of the plurality of cuts.

13. The medical device as claimed in claim 9, wherein the longitudinal uncut width is between 0.01 mm and 0.03 mm between each of the plurality of cuts, and wherein the pre-shaped segment transitions from the first geometric configuration to the second geometric configuration under a load between 5 gF and 7 gF. [26]

14. The medical device of claim 13, wherein the cut pattern defines a strut height between 0.06 mm and 0.15 mm between each of the plurality of cuts.

15. The medical device of claim 13 or 14, wherein the cut pattern defines a pitch between 0.05 mm and 0.07 mm for each of the plurality of cuts.

16. The medical device of claim 13, 14 or 15, wherein the catheter body includes a first tube segment proximal of the distal segment, the first tube segment having a cut pattern therein defining: a plurality of cuts forming an interrupted spiral cut, with each cut having a width between 0.03 mm and 0.05 mm, a longitudinal uncut width between 0.03 mm and 0.04 mm between each of the plurality of cuts, and a strut height between 0.08 mm and 0.12 mm between each of the plurality of cuts.

17. The medical device of claim 16, wherein the cut pattern of the first tube segment defines a pitch between 0.06 mm and 0.09 mm for each of the plurality of cuts.

18. The medical device of claim 16, wherein the catheter body includes a second tube segment proximal of the first tube segment, the second tube segment having a cut pattern therein defining: a plurality of cuts forming an interrupted spiral cut, with each cut having a width between 0.03 mm and 0.05 mm, a longitudinal uncut width between 0.06 mm and 0.07 mm between each of the plurality of cuts, and a strut height between 0.08 mm and 0.12 mm between each of the plurality of cuts.

19. The medical device of claim 18, wherein the cut pattern of the second tube segment defines a pitch between 0 .09 mm and 0.11 mm for each of the plurality of cuts.
